# EUROPEAN PATENT APPLICATION

(11) **EP 1 618 800 A1**
(43) Date of publication of application: **25.01.2006**
(21) Application number: 04017580.4
(22) Date of filing: 24.07.2004
(51) Int. Cl.: A23L 1/30, A23L 1/307, A23C 9/13

(54) **Active compositions comprising lycopene, cytidin and fatty acids**

(71) Applicant: Cognis IP Management GmbH, 40589 Düsseldorf (DE)
(72) Inventor: Fabry, Bernd, Dr., 41352 Korschenbroich (DE); Rull Prous, Santiago, 08034 Barcelona (ES)

(57) **Abstract**

Suggested are active compositions, comprising
(a) extracts of tomatoes or their active principles, and
(b) physiologically active fatty acids, their salts or their esters.

## Description

### Object of the invention

The present invention refers to the area of food additives and more particularly relates to active compositions, for both oral or topical administration, and their use for making food, cosmetic, or pharmaceutical compositions.

### State of the art

The tomato may be one of the most perfect vegetables on earth (technically, it is a fruit/berry). Its secret lies in its content of lycopene, which is responsible for the tomato's red colour, but more importantly than that, it possesses extraordinary anti-carcinogenic and anti-tumour powers. According to recent studies, lycopene may be an even more effective substance than beta-carotene in protecting humans against lung cancer. Studies at Johns Hopkins University showed a severe lack of lycopene in the blood of those patients with pancreatic, rectal, and bladder cancer, and researchers at the University of Illinois found low lycopene levels in the blood of women with a pre-cancerous condition called cervical intro-epithelial neoplasma. Moreover, it is known that lycopene has twice the punch of beta-carotene in quenching oxygen radicals which are implicated in the initiation of tumour cells. Moreover, it is reported that an optimal absorption of carotenoids, being typically lipid-soluble chemicals, is improved in the presence of a small amount of dietary oil or fat. Research in the field of nutrition and health has shown that mono-saturated oils, such as olive oil, are most desirable, since such oils do not increase the risk of arteriosclerosis, coronary heart disease, or nutritionally linked cancers. Finally, according to the teaching of international patent application **WO 99/055350 A1** (Rowett), extracts of fruit juices, in particular extracts of tomatoes, are suggested for use in the prophylaxis or treatment of a medical condition initiated or characterised by platelet aggregation.

Although tomatoes in general and more particularly their active principles, e.g., lycopene can be considered to behave as some kind of "wonder weapon" for fighting a multitude of diseases, there is still a need for increasing their activity, for example, by identifying further active agents with synergistic properties.

Therefore, the problem which underlies the present invention can be regarded as the need for developing new active compositions based on tomato extracts or their active principles which show an improved behaviour with respect to anti-oxidative power, cancer protection, and platelet aggregation.

### Description of the invention

The present invention claims are active compositions, comprising
(a) extracts of tomatoes or their active principles, and
(b) physiologically active fatty acids, their salts or their esters.

Surprisingly it has been found that the addition of physiologically active fatty acids or their derivatives, for example, conjugated linoleic acid or unsaturated fish fatty acids increases the advantageous properties of tomato extracts rich in lycopene in a synergistic manner. Especially it has been found that mixtures according to the present invention show a much higher potential for trapping UV radicals as the tomato extracts themselves. The compositions may be applied orally, for example, by adding the fatty compound (b) to a tomato juice or a tomato ketchup, or topically, for example, by incorporation into a skin cream or lotion.

### Extracts of tomatoes

Tomato extracts, and in particular aqueous extracts of tomato representing component (a), have been found to contain lycopene (β,β-carotin) as a major constituent and in addition a mixture of nucleosides, preferably cytidine. According to the technical teaching of the present invention it has been found that the combination of lycopene and said nucleosides are necessary to achieve the proposed effects.

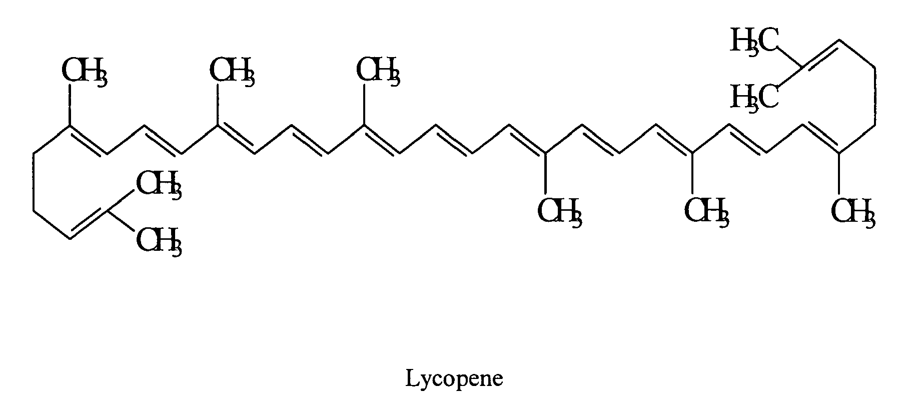

The extracts of the invention can be prepared by homogenising the flesh of a, preferably peeled, fruit and then by removing solids therefrom, for example, by means of centrifugation. Thus the extract is typically an aqueous extract which can consist essentially of the juice of the fruit, optionally with the addition of extra water during the homogenising step. Such aqueous extracts can be concentrated, enriched or condensed by, for example, standard techniques, e.g. evaporation under reduced pressure. Examples of concentrates are those which are at least 2-fold concentrated, more usually, at least 4-fold, for example, at least 8f old, or at least 40-f old, or at least 1 00-f old, or at least 200-f old, or at least 1000fold.

The extracts can be fractionated to isolate one or more active fractions therein by, for example, molecular weight filtration or chromatography on a suitable solid support, such as a sepharose gel (for size exclusion chromatography) or ion exchange column using HPLC on a suitably treated silica or alumina, for example, ODS coated silica; or by solvent extraction. Experiments carried out on tomato extracts have revealed that the active component(s) of the extract passes through an ultra-filtration filter having a molecular weight cut-off of 1000, and is colourless or straw-coloured, water soluble, and does not lose significant activity when boiled.

Alternatively, the extracts or active fractions thereof can be dried, e.g. by spray drying or freeze drying, and the dried product formulated in a solid or semi solid dosage form, for example, as a tablet, lozenge, capsule, powder, granulate, or gel. Instead, simple dried extracts can be prepared without any additional components. Alternatively, dried extracts can be prepared by adsorbing onto a solid support; for example, a sugar, such as sucrose, lactose, glucose, fructose, mannose, or a sugar alcohol, such as xylitol, sorbitol, or mannitol; or a cellulose derivative. Other particularly useful adsorbents include starch-based adsorbents, such as cereal flours, for example, wheat flour and corn flour. For tablet formation, the dried extract is typically mixed with a diluent, such as a sugar, e.g., sucrose and lactose, and sugar alcohols, such as xylitol, sorbitol and mannitol; or modified cellulose or cellulose derivative, such as powdered cellulose, or microcrystalline cellulose, or carboxymethyl cellulose. The tablets will also typically contain one or more excipients selected from granulating agents, binders, lubricants, and disintegrating agents. Examples of disintegrants include starch and starch derivatives, and other swellable polymers, for example, crosslinked polymeric disintegrants, such as cross-linked carboxymethylcellulose, crosslinked polyvinylpyrrolidone, and starch glycolates. Examples of lubricants include stearates, such as magnesium stearate and stearic acid. Examples of binders and granulating agents include polyvinylpyrolliclone. Where the diluent is naturally not very sweet, a sweetener can be added, for example, ammonium glycyrrhizinate or an artificial sweetener, such as aspartame or sodium saccharinate. Dried extracts can also be provided in a powder form for incorporation into snack food bars, for example, fruit bars, nut bars, and cereal bars. For presentation in the form of snack food bars, the dried extracts can be admixed with any one or more ingredients selected from dried fruits, such as sun-dried tomatoes, raisins, and sultanas, ground nuts, or cereals, such as oats and wheat.

Dried extracts can be provided in a powder form for reconstitution as a solution. As such they can also contain soluble excipients, such as sugars, buffering agents, such as citrate and phosphate buffers, and effervescent agents formed from carbonates, e.g., bicarbonates, such as sodium or ammonium bicarbonate, and a solid acid, for example, citric acid or an acid citrate salt. In one preferred embodiment, dried extract is provided in powder form optionally together with a preferred solid (e.g., powdered) excipient for incorporation into capsules, for example, a hard gelatine capsule. A solid or semisolid dosage form of the present invention can contain up to about 1.000 mg of the dried extract, for example, up to about 800 mg.

### Physiologically active fatty acids, their salts and their esters

A common criteria for fatty acids with physiological activity, which represent component (b), is a fat chain having a sufficient number of carbon atoms providing a lipophilic behaviour that allows the molecule to pass through the gastrointestinal tract of the body and having a sufficient number of double bonds. Therefore, said fatty acids usually comprise 18 to 26 carbon atoms and 2 to 6 double bonds.

In a first embodiment of the present invention conjugated linoleic acid (CLA) or its alkaline or alkaline earth salts and esters, preferably, their calcium salts and their esters with lower aliphatic alcohols having 1 to 4 carbon atoms - or their glycerides, specially their triglycerides come into account. Conjugated linoleic acid (CLA) represents a commercially available product which usually is obtained by base-catalysed isomerisation of sunflower oil or their respective alkyl esters and subsequent isomerisation in the presence of enzymes. CLA is an acronym used for positional and geometric isomers deriving from the essential fatty acid linoleic acid (LA, cis-9,cis-12-octadecadienoic acid, 18:2n-6). From a physiological point of view the use of the *cis-*9,*trans-*11 isomer according to the present invention is of special importance having at least 30, preferably at least 50, and most preferably at least 80 % b.w. of said *cis-*9,*trans-*11 isomer - based on the total CLA content of the crude mixture. In addition, it has been found advantageous if the content of the *trans-*10,*cis-*12 isomer is at most 45, preferably at most 10 % b.w. and most preferably less than 1 % b.w., and the sum of 8,10-, 11,13- and *trans,trans-*isomers in total is less than 1 % b.w. - again based on the total CLA content. Such products can be found in the market, for example, under the trademark Tonalin® CLA-80 (Cognis).

In a second embodiment also so-called omega-3 fatty acids can come into account, which typically comprise 18 to 26, preferably 20 to 22 carbon atoms and at least 4 and up to 6 double bonds. Also these molecules are very well known from the art and can be obtained by standard methods of organic chemistry, for example, via transesterification of fish oils, followed by urea precipitation of the alkyl esters thus obtained and a final extraction using non-polar solvents as described in the German patent **DE 3926658 C2** (Norsk Hydro). Fatty acids thus obtained are rich in omega-3 (all-Z)-5,8,11,14,17-eicosapentanoic acid (EPA) C 20 : 5 and (all-Z)-4,7,10,13,16,19-docosahexanoic acid (DHA) C 22 : 6. Such products can be found in the market under the trademark Omacor® (Pronova).

In a third embodiment also linoleic acid, vaccinic acid (trans 11-octadecenoic acid), or cishexadecenoic acid (obtained, for example, from the plant *Thunbergia alata*) can be used.

In addition, said physiologically active fatty acid esters can not only be used in form of their lower alkyl esters or glycerides. An additional well preferred embodiment of the present invention relates to compositions comprising esters of said fatty acids with sterols. Like glycerides, sterol esters are easily resorbed and split by the human body. However, a significant advantage comes from the fact that the cleavage of the ester bond releases a second molecule with health promoting properties. To avoid unclarities, the phrases "sterol", "stanol", and "sterin" shall be used as synonyms defining steroids showing a single hydroxyl group linked to the C-3. In addition, sterols which consist of 27 to 30 carbon atoms, may show a double bond, preferably in 5/6 position. According to the present invention, esters of CLA or omega-3 fatty acids with β-sitosterol or its hydrogenation product β-sitostanol are preferred.

### Plant extracts

In a preferred embodiment of the present invention the new active composition may comprise further plant extracts as an additional component (c). Typically, said plant extracts are chosen from the plants selected from the group consisting of *Ginkgo biloba, Camellia sinensis, Oleacea europensis, Glyzyrrhiza glabra, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Valeriana officinalis, Castanea sativa, Salix alba* and *Hapagophytum procumbens.* In the following a short summary of the composition and the main constituents of the cited extracts is given.

### • Ginkgo biloba

The active ingredients of extracts from the leaves of the ginkgo tree *(Ginkgo biloba)* are flavonoid glycosides, which among others contain (iso)quercitin glycosides, kaempferol, kaempferol-3-rhamnosides, isorhamnetin, luteoline glycosides, sitosterol glycosides, and predominantly hexacyclic terpene lactones, consisting of ginkgolides A, B, C, J, M, and bilobalides.

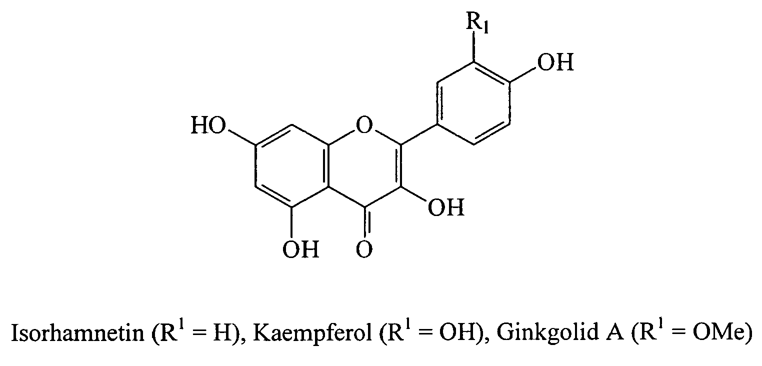

### • Camellia sinensis

Leaves of green tea contain many compounds, such as polysaccharides, volatile oils, vitamins, minerals, purines, alkaloids (e.g., caffeine), and polyphenols (catechins and flavonoids). Although all three tea types have antibacterial and free radical capturing (antioxidising) activities, the efficacy decreases substantially the darker the variety of tea is. This is due to the lower content of anti-oxidising polyphenols remaining in the leaves. Among the various components of green tea extracts, polyphenols of the flavonoid and catechin type ("tea tannins") are the most important ones.

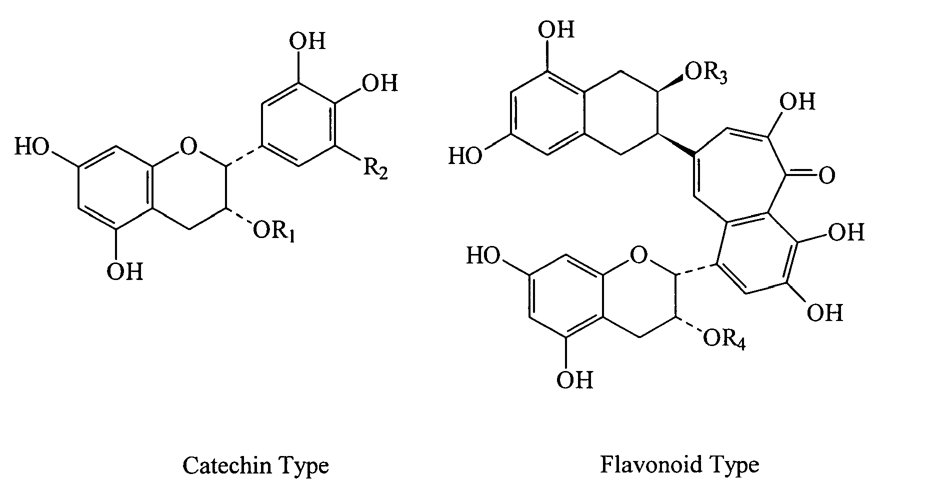

| | R1 | R2 | R3 | R4 |
|---|---|---|---|---|
| (-)-Epicatechin | H | H | | |
| (-) Epigallocatechin | H | OH | | |
| (-) Epicatechin gallate | Galloyl | H | | |
| (-) Epigallocatechin gallate | Galloyl | OH | | |
| Theaflavin | | | H | H |
| Theaflavin monogallate A | | | Galloyl | H |
| Theaflavin monogallate B | | | H | Galloyl |
| Theaflavin digallate | | | Galloyl | Galloyl |

### • Oleacea europensis

The main constituent of the leaves of the olive tree (*Oleacea europensis*) is the antioxidant oleuropein, which is also the main source for hydroxytyrosol.

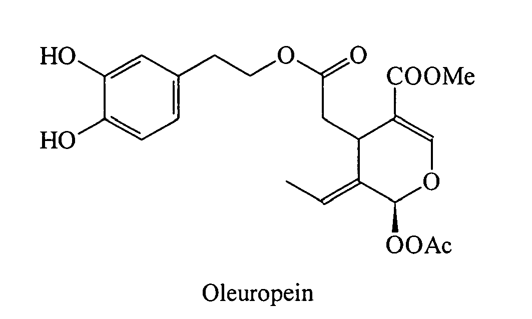

### • Glyzyrrhiza glabra

Main component of *Glyzyrrhiza glabra* is glycyrrhicinic acid:

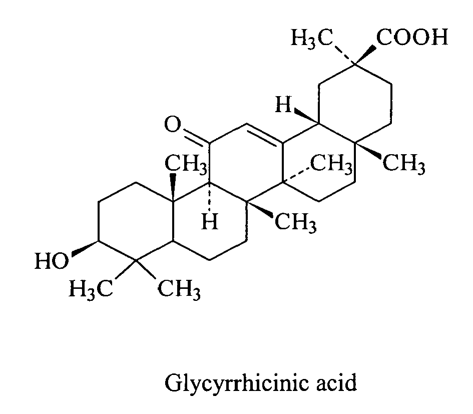

### • Vaccinium myrtillus

Extracts of blueberries (*Vaccinium myrtillus)* comprise a mixture of at least 15 different anthocyanosides, like the following.

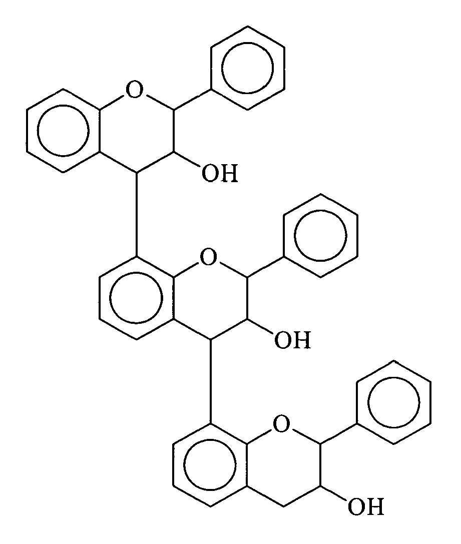

Usually, extracts of Vaccinium comprise from 20 to 25 % b.w. of anthocyanosides, from 5 to 10 % b.w. of tannins, and small amounts of various alkaloids like, e.g., myrtin and epimyrtin, phenolic acids, and glycosides of quercitrin, isoquercitrin, and hyperosid.

### • Trifolium pratense

The main active principles of red clover (*Triflolium pratense*) are isoflavones, e.g., daidzein, genestein, formononentin, and biochanin as well as their glucosides like ononin or sissostrin:

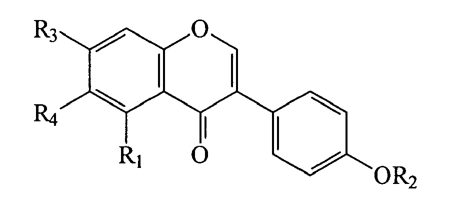

| Isoflavonglucosides | R₁ | R₂ | R₃ | R₄ |
|---|---|---|---|---|
| Daizidin | H | H | Glucose | H |
| Genistin | H | H | Glucose | OH |
| Ononin | H | CH₃ | Glucose | H |
| Sissostrin | H | CH₃ | Glucose | OH |

### • Litchi sinensis

Extracts of pericarps from Litchi *(Litchi sinensis)* are well known for their high content of flavon derivatives, e.g., 2-phenyl-4H-1-benzopyrans, flavanen, flavan-3-ols (catechins, catechin oligomeren), flavan-3,4-diols (leucoanthocyaniden), flavons, flavonols, and flavonons. The main components, however, represent condensed tannins, so-called procyanodols (OPC). These compounds comprise 2 to 8 monomers of the catechin or epicatechin-type, e.g., procyanidins, proanthocynidins, procyanidoel, oligoprocyanidins, leucoanthocyanidins, leucodelphinins, leucocyanins, and anthocyanogens. OPC, mainly the preferred proanthocyanidin A2 (OPC A2) behave like vitamin P, especially with respect to MMP inhibition.

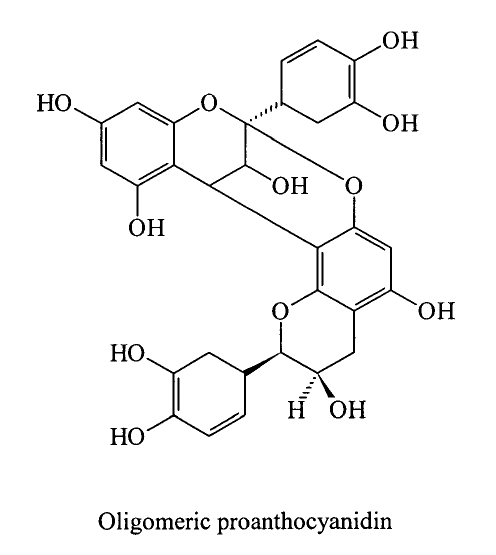

### • Vitis vinifera

The main actives of grape vine *(Vitis vinifera*) are polyphenols of the OPC type.

### • Brassica oleracea

The main active principles of cauliflower *(Brassica oleracea*) are amino acids, especially methionin and cystein, and glucosinolates, e.g., glucoraphanin.

### • Punica granatum

The main active principles of grenadine (*Punica granatum*) are sugars, citric acid, and delphinidin-1,2-glykoside or its aglykon.

### • Petroselinium crispum

The main constituent of the fatty oil of parsley *(Petroselinium crispum)* is petroselinic acid. The extracts, however, show high contents of apiol (1-allyl-2,5-dimethoxy-3,4-(methylendioxy)benzol), and in addition apiin, myristicin, pinen, and selinen.

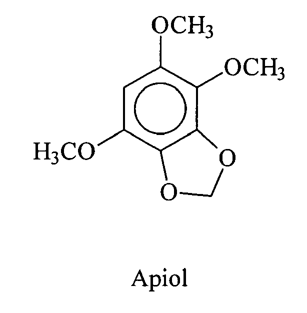

### • Centella asiatica

Main constituents of *Centella asiatica* are high condensed naphthenic acids, especially asiatica acid and madecassica acid and their glycosides.

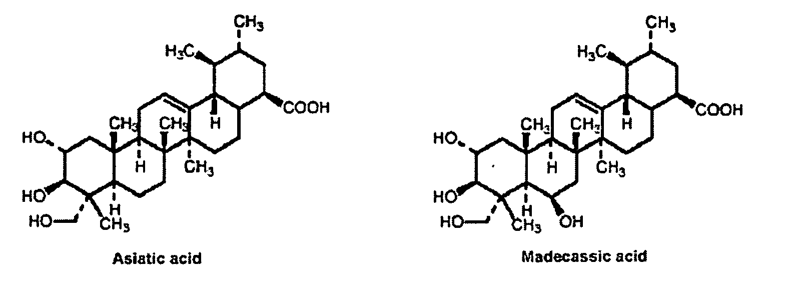

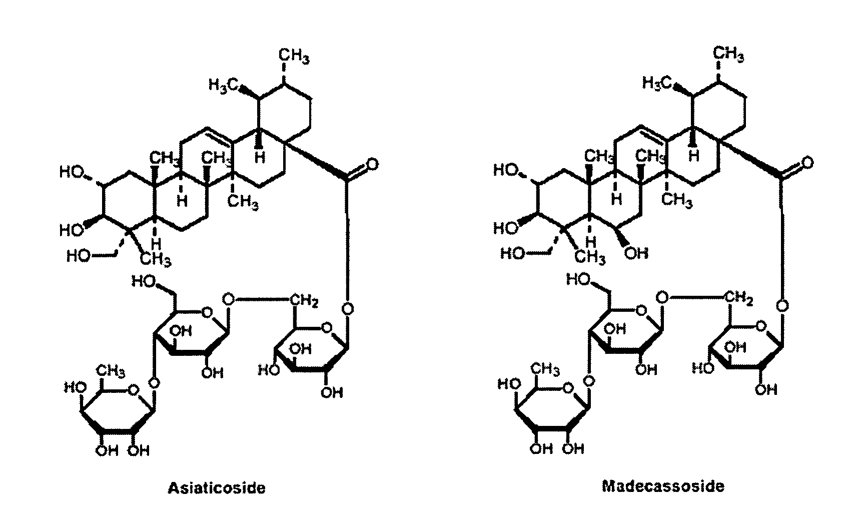

### • Passiflora incarnata

Extracts of passion flower (*Passiflora incarnata*) are rich in flavons of the apigenin and luteolin-type and their C-glycosides:

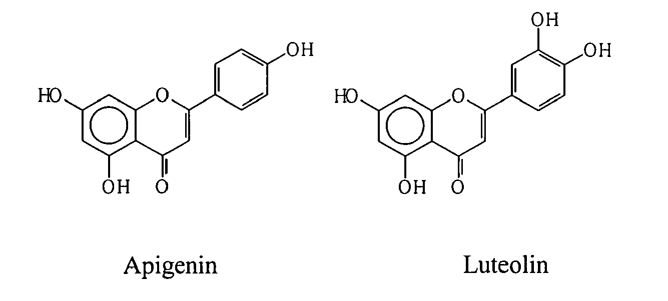

In addition, they comprise 2"-B-D-glucosides, schaftosides and iso-schaftosides, isovitexin, isoorientin, vicenin-2, incenin-2, daponanin, and trace elements like calcium, phosphor, and iron.

### • Medicago sativa

Extracts of Alfalfa *(Medicago sativa*) are rich in isoflavons, e.g., daidzein, genestein, formononetin, biochanin A, and tricin :

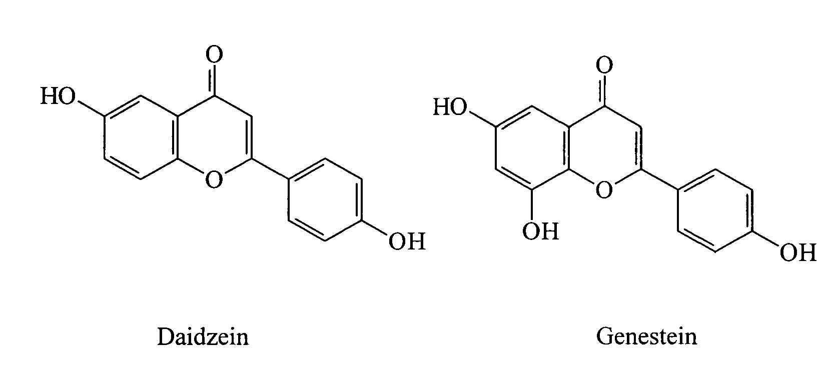

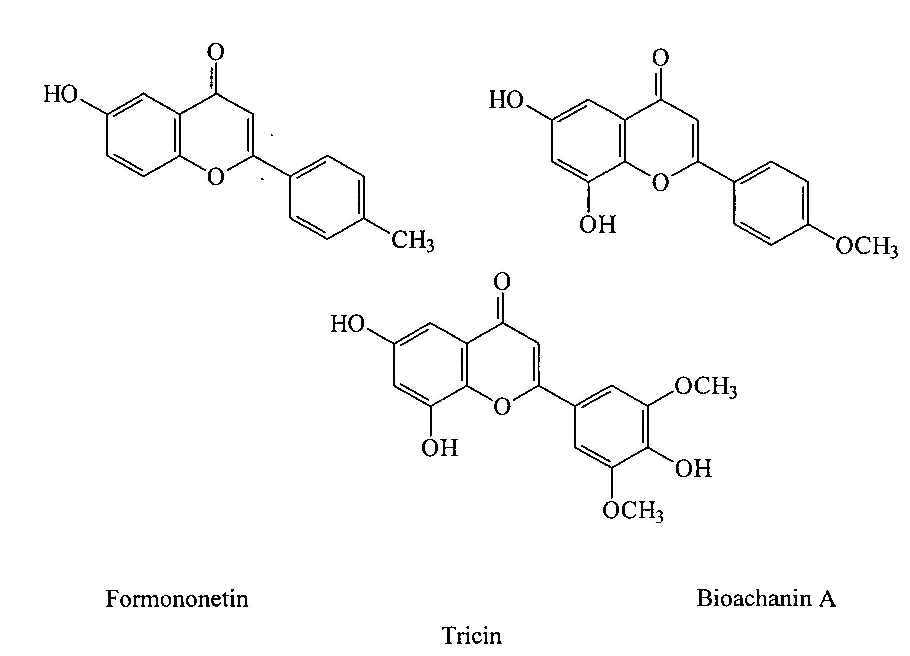

### • Valeriana officinalis

The main constituents of extracts of *Valeriana officinalis* are valeric acid, valerianone, and borneol esters.

### • Castanea sativa

The main ingredients of horse chestnuts *(Castanea sativa*) are saponins and escin, which is a mixture of two glycosides whose aglycons are derived from proteoescigenin, while the sugars represent either glucoronic acid or two molecules of D-glucose. Said glycosides differ in the acyl groups in the C22-position.

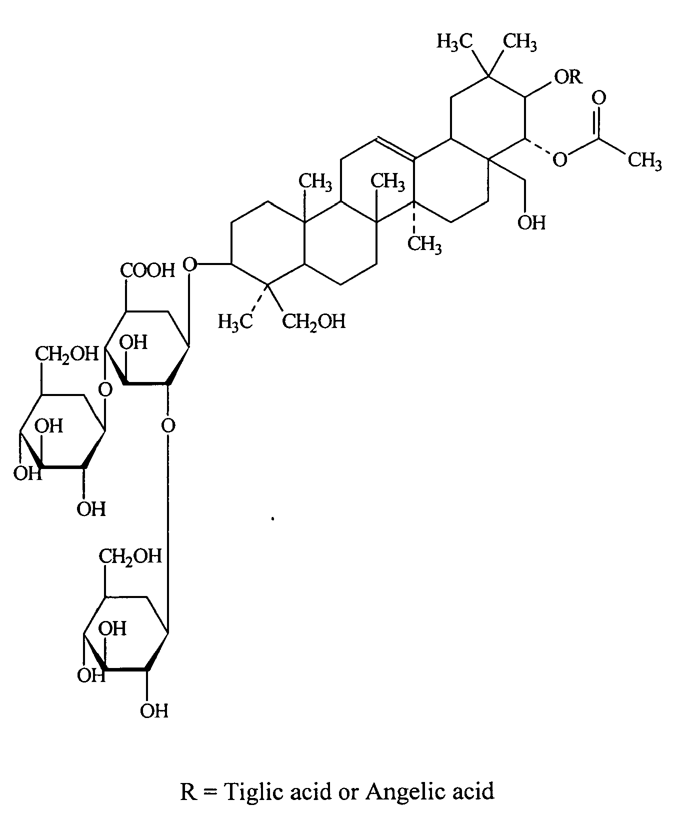

While α-escin represents an amorphous powder which melts between 225 and 227 °C and is easily soluble in water, β-escin (which is also called flogencyl) forms flakes which are practically water-insoluble but can be dissolved in alcohol.

### • Salix alba

Main constituents of *Salix alba* are phenolic glycosides and especially salicylates, e.g., salicin, salicortin, and tremulacin:

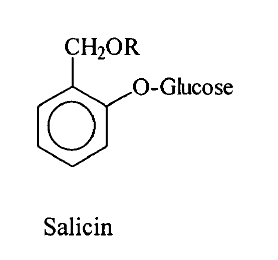

### • Harpagophytum procumbens

The main active principles of Devil's Claw *(Harpagophytum procumbens)* are iridoidglucosides, harpagosides, harpagides and procumbides.

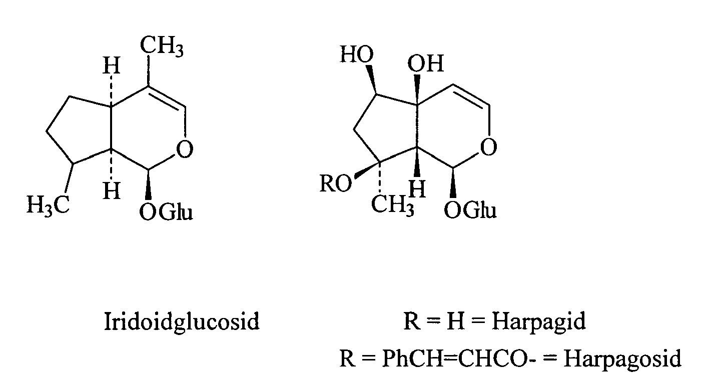

In addition, one finds stachylose, free and glycosylated phytosterols (e.g., β-sitosterol), flavonoides (e.g., kaempferol, luteolin), phenolic acids and glycosidic phenylpropanoicacid esters (e.g., verbacosides, isoacteosides).

### Active compositions

The compositions according to the present invention may be administered orally or topically to the human body. Usually, they comprise extracts and fatty acids in a weight ratio from 99 to 1 to 50 : 50 and more particularly from 95 : 10 to 75 : 25. The highest synergistic effects, however, are observed at ratios from 92 : 8 to 80 : 20. In general, the compositions can be used in a concentration of up to about 90, particularly from 10 to 75, and more particularly from 25 to 50 % b.w. - based on the final composition. The amount of plant extracts (component c) is typically from 1 to 10 and particularly from 2 to 5 % b.w. Typically, administration at least 100 mg, preferably 200 mg of the mixtures according to the invention - based on the active matter - per day to a human would lead to the proposed benefits and especially reduces the diseases linked to the interaction of free radicals with human cells.

### Encapsulation

Dried mixtures according to the present invention can also be formulated as powders, granules or semisolids for incorporation into capsules. When used in the form of powders, the compositions can be formulated together with any one or more excipients, or they can be presented in an undiluted form. For presentation in the form of a semisolid, the dried mixtures can be dissolved or suspended in a viscous liquid or semisolid vehicle, such as a polyethylene glycol, or a liquid carrier, such as a glycol, e.g., propylene glycol, or glycerol, or a vegetable or fish oil, for example, an oil selected from olive oil, sunflower oil, safflower oil, evening primrose oil, soya oil, cod liver oil, herring oil, etc. Such extracts can be macro-encapsulated, that means filled into capsules of either the hard gelatine or soft gelatine type or made from hard or soft gelatine equivalents, soft gelatine or gelatine-equivalent capsules preferred for viscous liquid or semisolid fillings.

In a special embodiment of the present invention said active compositions are micro-encapsulated. "Microcapsules" are understood to be spherical aggregates with a diameter from about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("micro-sponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third, etc. membrane. The membrane may consist of natural, semisynthetic or synthetic materials. Natural membrane materials are, for example, gum arabic, agar agar, agarose, maltodextrins, alginic acid and salts thereof, for example, sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are, inter alia, chemically modified celluloses, more particularly cellulose esters and ethers, for example, cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. Examples of known microcapsules are the following commercial products (the membrane material is shown in brackets) *Hallcrest Microcapsules* (gelatin, gum arabic), *Coletica Thalaspheres* (maritime collagen), *Lipotec Millicapseln* (alginic acid, agar agar), *Induchem Unispheres* (lactose, microcrystalline cellulose, hydroxypropylmethyl cellulose), *Unicerin C30* (lactose, microcrystalline cellulose, hydroxypropylmethyl cellulose), *Kobo Glycospheres* (modified starch, fatty acid esters, phospholipids), *Softspheres* (modified agar agar), *Kuhs Probiol Nanospheres* (phospholipids) and *Primaspheres* or *Primasponges* (chitosan, anionic polymers). The encapsulation of the compositions according to the present invention is preferred in case the actives are administered orally and should be liberated at a special part of the intestine. Therefore, a person skilled in the art can easily select the adequate encapsulation system by comparing the stability of the capsules under the pH-conditions of the respective part of the intestine.

### Industrial application

As explained above, due to its advantageous properties further objects of the present invention refer to food compositions, cosmetic compositions or pharmaceutical compositions comprising working amounts of said components (a) and (b). More particularly, said food composition is either a tomato juice, a tomato ketchup or a milk product (e.g., a yoghurt) and said cosmetic composition is a skin cream or lotion.

Finally, further objects of the present invention is the use of compositions comprising said components (a) and (b) for making food compositions, cosmetic compositions or pharmaceutical compositions, and especially for making a medicament for use as an anti-cancer or anti-thrombotic agent.

### Food compositions

As outlined above, the compositions according to the present invention can be formulated in a variety of ways. Preferably they are formulated for oral or buccal administration. As such, they can be formulated as solutions, suspensions, syrups, tablets, capsules, lozenges, snack bars, inserts, and patches by way of example. Such formulations can be prepared in accordance with methods well known per se. It is preferred that the formulations are low in, or substantially free of, lipid materials. For example, the compositions can be formed into syrups or other solutions for administration orally, for example, health drinks, in the presence of one or more excipients selected from sugars, vitamins, flavouring agents, colouring agents, preservatives, and thickeners. Tonicity adjusting agents, such as sodium chloride or sugars, can be added to provide a solution of a particular osmotic strength, for example, an isotonic solution. One or more pH adjusting agents, such as buffering agents can also be used to adjust the pH to a particular value, and preferably maintain it at that value. Examples for buffering agents include sodium citrate/citric acid buffers and phosphate buffers.

### Cosmetic and/or pharmaceutical preparations

In case the compositions according to the presentation invention are administered topically, that means directly to the skin, they may contain surfactants, emulsifiers, superfatting agents, pearlizing waxes, consistency factors, polymers, silicone compounds, waxes, stabilizers, primary and secondary sun protection agents, anti-dandruff agents, biogenic agents, film formers, swelling agents, hydrotropes, preservatives, solubilizers, complexing agents, reducing agents, alkalizing agents, perfume oils, dyes, and the like as additional auxiliaries and additives.

### Surfactants

Other preferred auxiliaries and additives are anionic and/or amphoteric or zwitterionic surfactants. Typical examples of anionic surfactants are soaps, alkyl benzenesulfonates, alkanesul-fonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids, such as, for example, acyl lactylates, acyl tartrates, acyl glutamates, and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homologue distribution although they preferably have a narrow-range homologue distribution. Typical examples of amphoteric or zwitterionic surfactants are alkylbetaines, alkylamidobetaines, aminopropionates, aminoglycinates, imidazolinium betaines, and sulfobetaines. The surfactants mentioned are all known compounds. Information on their structure and production can be found in relevant synoptic works, cf., for example, J. Falbe (ed.), "Surfactants in Consumer Products", Springer Verlag, Berlin, 1987, pages 54 to 124 or J. Falbe (ed.), "Katalysatoren, Tenside und Mineralöladditive (Catalysts, Surfactants and Mineral Oil Additives)", Thieme Verlag, Stuttgart, 1978, pages 123-217. The percentage content of surfactants in the preparations may be from 0.1 to 10% by weight and is preferably from 0.5 to 5% by weight, based on the preparation.

### Emulsifiers

Other surfactants may also be added to the preparations as emulsifiers, including for example:
- products of the addition from 2 to 30 mol of ethylene oxide and/or from 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products from 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products from 15 to 60 mol of ethylene oxide onto castor oil and/or hydrogenated castor oil;
- polyol esters and, in particular, polyglycerol esters, such as, for example, polyglycerol polyricinoleate, polyglycerol poly-12-hydroxystearate or polyglycerol dimerate isostearate. Mixtures of compounds from several of these classes are also suitable;
- addition products from 2 to 15 mol of ethylene oxide onto castor oil and/or hydrogenated castor oil;
- partial esters based on linear, branched, unsaturated, or saturated C_{6/22} fatty acids, ricinoleic acid and 12-hydroxystearic acid and glycerol, polyglycerol, pentaerythritol, - dipentaerythritol, sugar alcohols (for example, sorbitol), alkyl glucosides (for example, methyl glucoside, butyl glucoside, lauryl glucoside) and polyglucosides (for example, cellulose);
- mono-, di and trialkyl phosphates, and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols, and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

### • Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride, and technical mixtures thereof which may still contain small quantities of triglyceride from the production process. Addition products from 1 to 30, and preferably from 5 to 10 mol of ethylene oxide onto the partial glycerides mentioned are also suitable.

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products from 1 to 30, and preferably from 5 to 10 mol of ethylene oxide onto the sorbitan esters mentioned are also suitable.

### • Polyglycerol esters

Typical examples of suitable polyglycerol esters are Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina® ), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) and Polyglyceryl Polyricinoleate (Admul® WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like, optionally reacted with from 1 to 30 mol of ethylene oxide.

Typical anionic emulsifiers are aliphatic C₁₂₋₂₂ fatty acids, such as palmitic acid, stearic acid, or behenic acid, for example, and C₁₂₋₂₂ dicarboxylic acids, such as azelaic acid or sebacic acid, for example.

Other suitable emulsifiers are zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, cocoacylaminopropyl dimethyl ammonium glycinate, and 2-alkyl-3-carboxymethyl-3-hydroxyethyl imidazolines containing 8 to 18 carbon atoms in the alkyl or acyl group, and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkyl-aminopropionic acids, and alkylaminoacetic acids containing around 8 to 18 carbon atoms in the alkyl group. Particularly preferred ampholytic surfactants are N-cocoalkylamino-propionate, cocoacylaminoethyl aminopropionate, and C_{12/18} acyl sarcosine.

### Superfatting agents

Superfatting agents may be selected from such substances as, for example, lanolin, lecithin, and also polyethoxylated or acylated lanolin and lecithin derivatives, polyol fatty acid esters, monoglycerides, and fatty acid alkanolamides, the fatty acid alkanolamides also serving as foam stabilizers.

### Consistency factors

The consistency factors mainly used are fatty alcohols or hydroxyfatty alcohols containing 12 to 22, and preferably 16 to 18, carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil® types (hydrophilic silicas), polysaccharides, more particularly xanthan gum, guar-guar, agar-agar, alginates, and tyloses, carboxymethyl cellulose and hydroxyethyl cellulose, also relatively high molecular weight polyethylene glycol monoesters and diesters of fatty acids, polyacrylates (for example, Car-bopols® [Goodrich] or Synthalens® [Sigma]), polyacrylamides, polyvinyl alcohol and polyvinyl pyrrolidone, surfactants, such as, for example, ethoxylated fatty acid glycerides, esters of fatty acids with polyols, for example, pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives, such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers, such as, for example, Luviquat® (BASF), condensation products of polyglycols and amines, quaternized collagen polypeptides, such as, for example, Lauryldimonium Hydroxypropyl Hydrolyzed Collagen (Lamequat® L, Grünau), quaternized wheat polypeptides, polyethyleneimine, cationic silicone polymers, such as, for example, amodimethicone, copolymers of adipic acid and dimethylaminohy-droxypropyl diethylenetriamine (Cartaretine® , Sandoz), copolymers of acrylic acid with dimethyl diallyl ammonium chloride (Merquat® 550, Chemviron), polyaminopolyamides and crosslinked water-soluble polymers thereof, cationic chitin derivatives, such as, for example, quaternized chitosan, optionally in microcrystalline distribution, condensation products of dihaloalkyls, for example, dibromobutane, with bis-dialkylamines, for example, bis-dimethylamino-1,3-propane, cationic guar gum, such as, for example, Jaguar® CBS, Jaguar® C-17, Jaguar® C-16 of Celanese, quaternized ammonium salt polymers, such as, for example, Mirapol® A-15, Mirapol® AD-1, Mirapol® AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers, and optionally derivatized cellulose ethers and silicones.

### Pearlizing waxes

Suitable pearlizing waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing from 6 to 22 carbon atoms, especially long-chain esters of tartaric acid; fatty compounds, such as, for example, fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing from 12 to 22 carbon atoms with fatty alcohols containing from 12 to 22 carbon atoms and/or polyols containing from 2 to 15 carbon atoms and from 2 to 10 hydroxyl groups and mixtures thereof.

### Silicones

Suitable silicone compounds are, for example, dimethyl polysiloxanes, methylphenyl polysiloxanes, cyclic silicones, and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length from 200 to 300 dimethylsiloxane units and hydrogenated silicates. A detailed overview of suitable volatile silicones can be found in **Todd et al. in Cosm. Toil. 91, 27 (1976).**

### Waxes

Besides natural oils used, waxes may also be present in the preparations, more especially natural waxes, such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes, and microwaxes; chemically modified waxes (hard waxes), such as, for example, montan ester waxes, sasol waxes, hydrogenated jojoba waxes, and synthetic waxes, such as, for example, polyalkylene waxes and polyethylene glycol waxes.

### Stabilizers

Metal salts of fatty acids, such as, for example, magnesium, aluminium and/or zinc stearate or ricinoleate may be used as stabilizers.

### Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example, heat. UV-B filters can be oil-soluble or water-soluble. The following are examples of oil-soluble substances:
- 3-benzylidene camphor or 3-benzylidene norcamphor and derivatives thereof, for example, 3-(4-methylbenzylidene)-camphor;
- 4-aminobenzoic acid derivatives, preferably 4-(dimethylamino)-benzoic acid-2-ethylhexyl ester, 4-(dimethylamino)-benzoic acid-2-octyl ester and 4-(dimethylamino)-benzoic acid amyl ester;
- esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
- esters of salicylic acid, preferably salicylic acid-2-ethylhexyl ester, salicylic acid-4-isopropylbenzyl ester, salicylic acid homomenthyl ester;
- derivatives of benzophenone, preferably 2-hydroxy-4-methoxybenzophenone, 2-hydroxy-4-methoxy-4'-methylbenzophenone, 2,2'-dihydroxy-4-methoxybenzophenone;
- esters of benzalmalonic acid, preferably 4-methoxybenzalmalonic acid di-2-ethylhexyl ester;
- triazine derivatives, such as, for example, 2,4,6-trianilino-(p-carbo-2'-ethyl-1'-hexyloxy)-1,3,5-triazine and Octyl Triazone or Dioctyl Butamido Triazone (Uvasorb® HEB);
- propane-1,3-diones, such as, for example, 1-(4-tert.butylphenyl)-3-(4'-methoxyphenyl)-propane-1,3-dione;
- ketotricyclo(5.2.1.0)decane derivatives.

Suitable water-soluble substances are
- 2-phenylbenzimidazole-5-sulfonic acid and alkali metal, alkaline earth metal, ammonium, alkylammonium, alkanolammonium, and glucammonium salts thereof;
- sulfonic acid derivatives of benzophenones, preferably 2-hydroxy-4-methoxybenzophenone-5-sulfonic acid, and salts thereof;
- sulfonic acid derivatives of 3-benzylidene camphor, such as, for example, 4-(2-oxo-3-bornylidenemethyl)-benzene sulfonic acid and 2-methyl-5-(2-oxo-3-bomylidene)-sulfonic acid, and salts thereof.

Typical UV-A filters are, in particular, derivatives of benzoyl methane, such as, for example, 1-(4'-tert.butylphenyl)-3-(4'-methoxyphenyl)-propane-1,3-dione, 4-tert.butyl-4'-methoxydibenzoyl methane (Parsol® 1789) or 1-phenyl-3-(4'-isopropylphenyl)-propane-1,3-dione, and the enamine compounds (BASF). The UV-A and UV-B filters may of course also be used in the form of mixtures. Particularly favourable combinations consist of the derivatives of benzoyl methane, for example, 4-tert.butyl-4'-methoxydibenzoyl methane (Parsol® 1789) and 2-cyano-3,3-phenylcinnamic acid-2-ethylhexyl ester (Octocrylene® ), in combination with esters of cinnamic acid, preferably 4-methoxycinnamic acid-2-ethylhexyl ester and/or 4-methoxycinnamic acid propyl ester, and/or 4-methoxycinnamic acid isoamyl ester. Combinations, such as these are advantageously combined with water-soluble filters, such as, for example, 2-phenylbenzimidazole-5-sulfonic acid, and alkali metal, alkaline earth metal, ammonium, alkylammonium, alkanolammonium, and glucammonium salts thereof.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example, glycine, histidine, tyrosine, tryptophane) and derivatives thereof, imidazoles (for example, urocanic acid) and derivatives thereof, peptides, such as D,L-carnosine, D-carnosine, L-carnosine and derivatives thereof (for example, anserine), carotinoids, carotenes (for example, □-carotene, □-carotene, lycopene) and derivatives thereof, chlorogenic acid and derivatives thereof, liponic acid and derivatives thereof (for example, dihydroliponic acid), aurothioglucose, propylthiouracil and other thiols (for example, thioredoxine, glutathione, cysteine, cystine, cystamine and glycosyl, N-acetyl, methyl, ethyl, propyl, amyl, butyl and lauryl, palmitoyl, oleyl, □-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example, butionine sulfoximines, homo-cysteine sulfoximine, butionine sulfones, penta-, hexa- and hepta-thionine sulfoximine) in very small compatible dosages, also (metal) chelators (for example, alpha-hydroxyfatty acids, palmitic acid, phytic acid, lactoferrine), alpha-hydroxy acids (for example, citric acid, lactic acid, malic acid), humic acid, bile acid, bile extracts, bilirubin, biliverdin, EDTA, EGTA and derivatives thereof, unsaturated fatty acids and derivatives thereof (for example, linoleic acid, oleic acid), folic acid and derivatives thereof, ubiquinone and ubiquinol and derivatives thereof, vitamin C and derivatives thereof (for example, ascorbyl palmitate, Mg ascorbyl phosphate, ascorbyl acetate), tocopherols and derivatives (for example, vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihy-droguaiaretic acid, trihydroxybutyrophenone, uric acid and derivatives thereof, mannose and derivatives thereof, superoxide dismutase, zinc and derivatives thereof (for example, ZnO, ZnSO₄), selenium and derivatives thereof (for example, selenium methionine), stilbenes and derivatives thereof (for example, stilbene oxide, trans-stilbene oxide) and derivatives of these active substances suitable for the purposes of the invention (salts, esters, ethers, sugars, nucleotides, nucleosides, peptides, and lipids).

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example, prune extract, bambara nut extract, and vitamin complexes.

### Film formers

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof, and similar compounds.

### Hydrotropes

In addition, hydrotropes, for example, ethanol, isopropyl alcohol or polyols, may be used to improve flow behavior. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols, such as, for example, ethylene glycol, diethylene glycol, propylene glycol, butylene glycol, hexylene glycol and polyethylene glycols with an average molecular weight from 100 to 1000 dalton;
- technical oligoglycerol mixtures with a degree of self-condensation from 1.5 to 10, such as, for example, technical diglycerol mixtures with a diglycerol content from 40 to 50% by weight;
- methylol compounds, such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example, methyl and butyl glucoside;
- sugar alcohols containing 5 to 12 carbon atoms, for example, sorbitol or mannitol,
- sugars containing 5 to 12 carbon atoms, for example, glucose or sucrose;
- amino sugars, for example, glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid, and the other classes of compounds listed in Appendix 6, Parts A and B of the Kosmetikverordnung ("Cosmetics Directive").

### Complexing agents

The complexing agents used may be selected from EDTA, NTA, phosphonic acids, Triton B, turpinal and phenacetin. In addition, reducing agents, such as, for example, ascorbic acid, sodium sulfate, sodium thiosulfate, and the like may be present. Suitable alkalizing agents are ammonia, monoethanolamines, (L) arginine, AMP, etc.

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example, civet and beaver, may also be used. Typical synthetic perfume compounds are products of the ester, ether, aldehyde, ketone, alcohol, and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate, and benzyl salicylate. Ethers include, for example, benzyl ethyl ether while aldehydes include, for example, the linear alkanals containing from 8 to 18 carbon atoms, citral, citronellal, citronellyloxyacetaldehyde, cyclamen aldehyde, hydroxycitronellal, lilial, and bourgeonal. Examples of suitable ketones are the ionones, □-isomethylionone and methyl cedryl ketone. Suitable alcohols are anethol, citronellol, eugenol, isoeugenol, geraniol, linalool, phenylethyl alcohol, and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil, and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl, and floramat.

### Dyes

Suitable dyes are any of the substances suitable and approved for cosmetic purposes as listed, for example, in the publication **"Kosmetische Färbemittel"** of the Farbstoffkommission der Deutschen Forschungsgemeinschaft, **Verlag Chemie, Weinheim, 1984, pages 81 to 106**. Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800), and madder lake (C.I. 58000). Luminol may also be present as a luminescent dye. These dyes are normally used in concentrations from 0.001 to 0.1% by weight, based on the mixture as a whole.

The total percentage content of auxiliaries and additives may be from 1 to 50% by weight and is preferably from 5 to 40% by weight, based on the particular composition. The compositions may be produced by standard hot or cold processes.

### Examples

### Examples 1 and 2, Comparative examples C1 and C2

### Activity towards free radicals

In a first series of tests, the effectiveness of the extracts against oxidative stress was investigated by chemical methods. A commercially available extract of tomatoes, CLA (Tonalin® CLA, Cognis) and their mixtures were used in concentrations of 0.3 % b.w. The first test substrate selected was diphenyl picryl hydrazyl (DPPH), a purple-red coloured stable radical which changes into its colourless leuco derivative on contact with radical trappers. The change of colour was followed photometrically at 513 nm. The test results are set out in Table 1 ("DPPH Test") where the inhibition of DPPH is shown in % absolute.

In a second test, the hydroxylation of salicylic acid by hydroxyl radicals (from the reaction of hydrogen peroxide with iron(III) ions and EDTA) was investigated as a reference system. This reaction can also be photometrically investigated because the hydroxylation product is reddish in colour. The influence of the extracts on the formation of hydroxysalicylic acid was measured at an optical density of 490 nm. The results are also set out in Table 1 where inhibition is again shown in % absolute ("Salicylic Acid Test").

**Table 1 Radical-trapping properties in % absolute (results are averages of 2 measurements)**

| **Ex.** | **Sample** | **DPPH Test** | **Salicylic Acid Test** | |
|---|---|---|---|---|
| | | Batch A | Batch A | Batch B |
| 0 | Control | 0 | 0 | 0 |
| C1 | Tomato extract | 68 | 22 | 23 |
| C2 | CLA | 18 | 5 | 5 |
| 1 | Tomato extract: CLA = 70 : 30 | 90 | 33 | 11 |
| 2 | Tomato extract: CLA = 80 : 20 | 92 | 61 | 43 |
| | IC50 in % [% by weight] | 0.0068 | 0.072 | 0.115 |

### Examples 3 and 4, Comparative examples C3 and C4

### Activity towards free radicals (II)

In the following test, deoxyribose was used as the test substance. In the presence of hydroxyl radicals formed from H₂O₂ in the presence of Fe²⁺ and EDTA, deoxyribose is oxidized. A pink-coloured substance is formed from the oxidized form by condensation with thiobarbituric acid. The optical density was determined at 532 nm and is dependent on the content of oxidized deoxyribose. A radical-trapping substance reacts with the hydroxyl radicals formed and reduces the formation of pink-coloured substances in this reaction mixture. In addition, the same reaction was carried out without EDTA to investigate complexing properties of the extract with Fe²⁺ ions. The concentration of the samples has been again 0.3 % b.w. The results are set out in Table 2.

**Table 2 Radical-trapping properties with desoxyribose in % absolute (results are averages of 2 measurements)**

| **Ex.** | **Sample** | **Reaction with EDTA** | | **Reaction without EDTA** | |
|---|---|---|---|---|---|
| | | Batch A | Batch B | Batch A | Batch B |
| 0 | Control | 0 | 0 | 0 | 0 |
| C3 | Tomato extract | 5 | 4 | 5 | 2 |
| C4 | CLA | 1 | 0 | 0 | 0 |
| 3 | Tomato extract : CLA = 70 : 30 | 16 | 10 | 19 | 8 |
| 4 | Tomato extract: CLA = 80 : 20 | 38 | 31 | 39 | 43 |
| | IC50 in % [% by weight] | 0.14 | 0.16 | 0.14 | 0.11 |

### Examples 5 and 6, Comparative examples C5 and C6

### Activity towards free radicals (III)

In the next test, the radical-trapping properties of the test samples were investigated using xanthine oxidase as the test system. Under oxidative stress, the enzyme converts purine bases, for example, adenine or guanine, into uric acid with intermediate formation of oxygen radicals which are spontaneously decomposed into H₂O₂ and oxygen by Superoxid-Dismutase (SOD). These Superoxid radicals can be detected and quantitatively determined by reaction with luminol or luminol and microperoxidase and by NBT via the luminescence and the optical density at 490 nm. The luminescence yield diminishes in the presence of substances with radical-trapping properties. The concentration of the test samples has been at 0.3 % b.w. again. The results are set out in Table 3 where the inhibition is shown in % absolute ("Luminol Test").

**Table 3 Test with xanthan oxidase in % absolute (results are averages of 2 measurements)**

| **Ex.** | **Sample** | **Luminol** | | **Luminol + Microperoxidase** | | **NBT** | |
|---|---|---|---|---|---|---|---|
| | | Batch A | Batch B | Batch A | Batch B | Batch A | Batch B |
| 0 | Control | 0 | 0 | 0 | 0 | 0 | 0 |
| C5 | Tomato extract | 22 | 25 | 41 | 44 | 10 | 11 |
| C6 | CLA | 3 | 3 | 5 | 7 | 1 | 1 |
| 5 | Tomato extract : CLA = 70 : 30 | 45 | 36 | 57 | 51 | 21 | 22 |
| 6 | Tomato extract : CLA = 80 : 20 | 92 | 71 | 94 | 98 | 46 | 53 |
| | IC50 in % [% by weight] | 0.0020 | 0.0020 | 0.0275 | 0.0295 | 0.1112 | 0.0932 |

The results set out in Tables 1 to 3 show that the mixtures of tomato extracts and CLA show a synergistic anti-radical effect.

### Example 7

### Ketchup composition

70 parts of a tomato paste were mixed with 0.5 parts of conjugated linoleic acid (Tonalin CLA, Cognis), 0.5 parts of salts and 29.5 parts of water.

### Example 8

### Yoghurt composition

Soy milk is added to 15-75 parts by volume of cow milk to make 100 parts of the mixture. The mixture is then pasteurised at about 90 °C for 15 seconds and then cooled. The cooled, pasteurised mixtures are then inoculated with 3 to 5 percent by volume of a yoghurt culture having 1:1 ratio of *Lactobacillus bulgaricus* and *Bifidobacterium adolescentis*. The incubation is carried out at about 42 °C. In about 2 hours thickening will occur. The fermentation is carried out for about 5.5 hours. The yoghurt compositions thus obtained is treated with 1 % of inulin and 1 % of a 9:1 mixture of tomato extract and CLA - both based on the amount of micro-organisms being present in the composition. The products have a firm consistency and a flavour like or substantially indistinguishable from that of a corresponding yoghurt composition using 100 percent of fresh cow milk. A small amount of citric acid can be added to the fermentation mixture to enhance the flavour of the final yoghurt composition. A suitable amount of citric acid is 0.5 percent based on the weight of the composition.

## Claims

1. Active compositions, comprising
(a) extracts of tomatoes or their active principles, and
(b) physiologically active fatty acids, their salts or their esters.

2. Active compositions, comprising
(a) mixtures of lycopene and cytidin, and
(b) physiologically active fatty acids, their salts or their esters.

3. Compositions according to claims 1 and/or 2, **characterised in that** said physiologically active fatty acids (component b) comprise from 18 to 26 carbon atoms and from 2 to 6 double bonds.

4. Compositions according to any of the claims 1 to 3, **characterised in that** component (b) represents esters of said fatty acids with glycerol or sterol.

5. Compositions according to any of the claims 1 to 4, **characterised in that** component (b) represents conjugated linoleic acid or omega-3 fatty acids.

6. Compositions according to any of the claims 1 to 5, **characterised in that** they comprise component (a) and (b) in weight ratios from 99 : 1 to 50 : 50.

7. Compositions according to any of the claims 1 to 6, **characterised in that** they comprise as additional components (c) one or more plant extracts or their active principles.

8. Compositions according to claim 7, **characterised in that** said plant extracts are selected from the group consisting of *Ginkgo biloba, Camellia sinensis, Oleacea europensis, Glyzyrrhiza glabra, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Valeriana officinalis, Castanea sativa, Salix alba* and *Hapagophytum procumbens.*

9. Compositions according to any of the claims 1 to 8, **characterised in that** said mixtures are macro- or micro-encapsulated.

10. Composition according to any of claims 1 to 9, **characterised in that** said composition is a food composition, a cosmetic composition, or a pharmaceutical composition.

11. Composition according to claim 10, **characterised in that** said food composition is a tomato juice or a tomato ketchup or a milk product.

12. Composition according to claim 11, **characterised in that** said cosmetic composition is a skin cream or lotion.

13. Use of a composition according to claims 1 and/or 2 for making food compositions, cosmetic compositions, or pharmaceutical compositions.

14. Use of a composition according to claim 1 and/or 2 for making a medicament for use as an anti-cancer or anti-thrombotic agent.
